# EUROPEAN PATENT SPECIFICATION

(11) **EP 4 489 972 B1**
(45) Date of publication and mention of the grant of the patent: **24.09.2025**
(21) Application number: 23708450.4
(22) Date of filing: 27.02.2023
(51) Int. Cl.: B41J 29/02, A61B 5/344, B41J 29/13, A47B 88/40, B41J 2/32

(54) **PRINTING MECHANISM FOR MEDICAL MONITORING DEVICE AND MEDICAL MONITORING DEVICE**
DRUCKMECHANISMUS FÜR MEDIZINISCHE ÜBERWACHUNGSVORRICHTUNG UND MEDIZINISCHE ÜBERWACHUNGSVORRICHTUNG
MÉCANISME D'IMPRESSION POUR DISPOSITIF DE SURVEILLANCE MÉDICALE ET DISPOSITIF DE SURVEILLANCE MÉDICALE

(30) Priority: 07.03.2022 CN 202210215705; 07.03.2022 CN 202220478538 U
(43) Date of publication of application: 15.01.2025
(73) Proprietor: Koninklijke Philips N.V., 5656 AG Eindhoven (NL)
(72) Inventor: YANG, Lei, 5656 AG Eindhoven (NL); TANG, Liang Yong, 5656 AG Eindhoven (NL)
(74) Representative: Philips Intellectual Property & Standards
(86) International application number: PCT/EP2023/054764
(87) International publication number: WO 2023/169845

(56) References cited:
- WO-A1-2006/066302
- WO-A1-2007/065191
- US-A- 4 804 239
- US-A- 5 746 528
- US-A1- 2001 016 138

## Description

### FIELD OF THE INVENTION

The present invention relates to a printing mechanism for a medical monitoring device and a medical monitoring device comprising such a printing mechanism.

### BACKGROUND OF THE INVENTION

A medical monitoring device such as a fetal monitor, an electrocardiograph, a defibrillator or an ultrasound diagnostic device generally comprises a main unit and a printing mechanism disposed in the main unit. In the case of the printing mechanism for a fetal monitor, for example, the laminated thermal print papers are usually housed in a print paper drawer. When the printing mechanism operates, a rubber platen driven by a stepper motor rotates at a preset speed to drag a print paper past a thermal printing head to record a curve characterizing fetal vital characteristics on thermal print paper. A prerequisite for accurately printing is the stability of a print paper drawer, which means that proper damping is required when the print paper drawer moves, and proper force is required to maintain the print paper drawer at a working position without any wobbling and any offset after the print paper drawer is put in place. In the existing printing mechanism, the drawer-shaped print paper drawer has protruding slide rails on both sides, and the slide rails cooperate with the pulleys on the medical monitoring device to allow the print paper drawer to slide back and forth. The outer edge of the slide rails on the print paper drawer and the wall of a housing of the medical monitoring device limit the position of the print paper drawer. As the print paper drawer needs to be pushed and pulled frequently, the outer edge of the slide rails on the print paper drawer and the wall of the housing of the medical monitoring device can not cooperate too tightly. Otherwise, the sliding damping is excessive when the print paper drawer is pushed and pulled. Not tight cooperation between the outer edge of the slide rails on the print paper drawer and the wall of the housing of the medical monitoring device leads to wobbling of the print paper drawer in the housing. When the medical monitoring device operates in a vertically hung state, the print paper drawer can't be naturally positioned at any position on the slide rails but slides directly and completely out of the housing of the medical monitoring device due to gravitational force. Further, when the print paper drawer moves, the sliding damping comes from the sliding friction between the outside wall of the print paper drawer and the inside wall of the housing of the medical monitoring device. However, since the outer edge of the slide rails on the print paper drawer and the wall of the housing of the medical monitoring device can not cooperate too tightly, the print paper drawer can't move stably so that it is difficult to control the sliding friction force and the user thus feels a poor controllability when pushing and pulling the print paper drawer.

Therefore, there is a need to improve the existing printing mechanism for the medical monitoring device.

Documents US2001/016138A1, US5746528A, WO2006/066302A1, WO2007/065191A1 and US4804239A represent relevant prior art for the present invention.

### SUMMARY OF THE INVENTION

It is an object of the present invention to provide a printing mechanism for a medical monitoring device which can overcome at least one of the above-mentioned disadvantages in the prior art.

According to an aspect of the present invention, there is provided a printing mechanism for a medical monitoring device comprising:
a support plate;
a movable frame assembly disposed movably on the support plate, the movable frame assembly comprising a frame; and
a print paper drawer for holding the print papers, the print paper drawer being mounted on the frame to move along with the movable frame assembly;
the printing mechanism further comprising:
   at least one set of spaced sleeves provided on a top surface of a body of the support plate in a movement direction of the movable frame assembly; and
   a guide rod passing through holes defined by each set of the spaced sleeves and secured to a bottom of the frame at two ends, the guide rod guiding the movable frame assembly and moving along with the movable frame assembly;
   wherein a damping bracket is provided between adjacent sleeves of each set of the spaced sleeves, the damping bracket contacts the guide rod and exerts an upward force onto the guide rod perpendicularly to a movement direction of the guide rod.

Preferably, two elongated openings may be formed between adjacent sleeves of each set of the spaced sleeves so as to define an elastic beam between the two elongated openings, and the damping bracket is provided on the elastic beam.

Preferably, the damping bracket may be V-shaped, U-shaped or semi-circular.

Preferably, a guide groove may be provided on the body at a portion where the guide rod passes but no sleeve is provided, and the guide rod is partially accommodated in the guide groove when it passes the guide groove.

Preferably, spaced through-holes may be formed in the body at a position where the guide groove is provided, the printing mechanism further comprises a friction strip comprising a planar-shaped carrier and a projecting pad formed at the center of the carrier, the pad passes through the through-hole from the bottom of the body and protrudes toward a top of the body so that the guide rod squeezes the pad when it comes into contact with the pad.

Preferably, a bottom surface of the holes defined by the sleeves facing the body may be open, and the pad passes through a through-hole below the sleeve.

Preferably, a hollow section may be formed below a top surface of the pad.

Preferably, the printing mechanism may further comprise:
a support arm disposed on the support plate, the support arm provided with a printing head thereon, the movable frame assembly further comprising a rubber platen mounted at a front end of the frame adjacent to the outside of the medical monitoring device to be in contact with the printing head under pressure; and
a driving mechanism for driving the rubber platen to rotate;
wherein the movable frame assembly is disposed between the support plate and the support arm.

Preferably, the printing mechanism may further comprise a locking mechanism comprising a pair of resilient clamping claws disposed opposite to each other at a rear end of the frame and a locking post disposed on the support plate correspondingly to the resilient clamping claws, the resilient clamping claws tend to clamp together, the resilient clamping claws open under an external pushing force to receive the locking post and clamp the locking post tightly, and the resilient clamping claws are disengaged from the locking post under an external pulling force.

Preferably, the driving mechanism may comprise a first pulley fixedly mounted at one end of the rubber platen, an electric motor mounted on the frame, a second pulley mounted on a rotating shaft of the electric motor, and a timing belt for connecting the first pulley and the second pulley so as to drive the rubber platen to rotate when the electric motor operates, and the electric motor is disposed at a rear end of the frame adjacent to the inside of the medical monitoring device.

Preferably, the support arm may be L-shaped, the support arm is rotatably mounted on the support plate at one end and provided with a printing head mounting portion at the other end, and the printing head is mounted on the printing head mounting portion.

Preferably, the frame may comprise a first mounting plate and a second mounting plate disposed opposite to each other, and a first connecting plate and a second connecting plate connecting fixedly the first mounting plate and the second mounting plate together, a first guide slot extending along a longitudinal direction is formed in the first mounting plate and a second guide slot extending along the longitudinal direction is formed in the second mounting plate, and two ends of the printing head mounting portion are movably supported in the first guide slot and the second guide slot.

Preferably, the medical monitoring device may be a fetal monitor.

According to another aspect of the present invention, there is provided a medical monitoring device, wherein the medical monitoring device comprises a printing mechanism for a medical monitoring device as above stated.

The printing mechanism for the medical monitoring device according to the present invention may provide a beneficial damping force for the movement of the movable frame assembly (i.e., the print paper drawer), which not only provides a good controllability when the user pushes and pulls the print paper drawer, but also prevents the print paper drawer from wobbling, thereby ensuring that the print paper drawer is positioned with high accuracy and is opened and closed smoothly when the printing mechanism operates in the horizontally arranged state or the vertically hung state.

### BRIEF DESCRIPTION OF THE DRAWINGS

FIG. 1 schematically shows in a perspective view a printing mechanism for a medical monitoring device according to the present invention;
FIG. 2 shows schematically in a perspective view a movable frame assembly of a printing mechanism for a medical monitoring device according to the present invention, wherein an electric motor and a rubber platen are mounted on the frame;
FIG. 3 shows schematically in a perspective view the printing mechanism for the medical monitoring device according to the present invention, wherein a support arm, a printing head mounting portion and a rubber platen have been removed to show clearly a print paper drawer;
FIG. 4 is a top view of the structure shown in FIG. 3;
FIG. 5 is a partially enlarged view of FIG. 4 showing a locking post to be clamped by resilient clamping claws;
FIG. 6 is a partially enlarged view of FIG. 4 showing a locking post clamped by resilient clamping claws in a locked state;
FIG. 7 schematically shows in a perspective view a support plate of a printing mechanism for a medical monitoring device according to the present invention;
FIG. 8 is a perspective view similar to that of FIG. 7, wherein two guide rods pass through sleeves provided on the support plate respectively;
FIG. 9 is a partially enlarged view of FIG. 8; and
FIG. 10 schematically shows in a perspective view a friction strip of a printing mechanism for a medical monitoring device according to the present invention.

### DETAILED DESCRIPTION OF THE EXEMPLARY EMBODIMENTS

Preferred embodiments of the present invention are described in detail hereinafter in connection with the drawings. It should be understood by those skilled in the art that the drawings are intended to illustrate the invention only and are not meant to form any limit to the present invention.

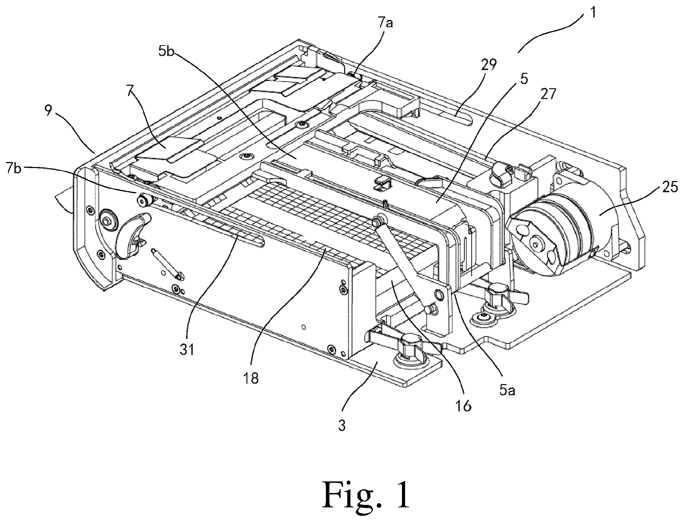

FIG. 1 schematically shows in a perspective view a printing mechanism for a medical monitoring device according to the present invention. As shown in FIG. 1, a printing mechanism 1 for a medical monitoring device according to the present invention generally comprises a support plate (or a base plate) 3 which is typically mounted on a housing of the medical monitoring device (not shown) such as a fetal monitor. The printing mechanism 1 for the medical monitoring device according to the present invention further comprises a support arm 5. In a preferred embodiment, the support arm 5 is substantially L-shaped, one end portion 5a of the support arm 5 is rotatably mounted on the support plate 3, and the other end 5b of the support arm 5 is provided with a printing head mounting portion 7 on which a printing head for example a thermal printing head is mounted. In Fig 1, the printing head is invisible because it is mounted on the underside of the printing head mounting portion 7. The printing head is electrically communicated with a main unit of the medical monitoring device by wire or wirelessly, and thus receives signals from the main unit to perform the printing operations. The printing mechanism 1 for the medical monitoring device according to the present invention also comprises a movable frame assembly 9, which is movably disposed on the support plate 3 and in a space between the support plate 3 and the support arm 5.

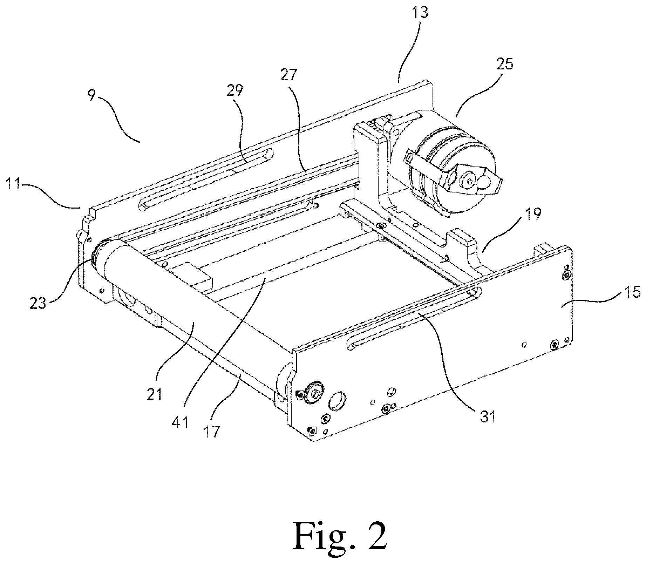

FIG. 2 shows schematically in a perspective view a movable frame assembly of a printing mechanism for a medical monitoring device according to the present invention, wherein an electric motor and a rubber platen are mounted on the frame. As shown in FIG. 2, the movable frame assembly 9 comprises a frame 11. The frame 11 comprises a first mounting plate 13 and a second mounting plate 15 disposed opposite to each other, and a first connecting plate 17 and a second connecting plate 19 that connect fixedly the first mounting plate 13 and the second mounting plate 15 together. In the preferred embodiment, the first mounting plate 13, the second mounting plate 15, the first connecting plate 17, and the second connecting plate 19 are metal members such as aluminum or other lightweight metal or alloy members, which are formed separately and then connected together by fasteners such as screws so that the frame 11 is formed as a highly rigid metal frame. However, it should be understood that the first mounting plate 13, the second mounting plate 15, the first connecting plate 17, and the second connecting plate 19 can also be cast in one piece from metal. Of course, the first mounting plate 13, the second mounting plate 15, the first connecting plate 17, and the second connecting plate 19 can also be molded in one piece from plastic. The movable frame assembly 9 also comprises a print paper drawer 16 mounted between the first mounting plate 13 and the second mounting plate 15 to hold the print papers 18. In FIG. 2, the print paper drawer 16 mounted on the frame 11 is not shown.

The movable frame assembly 9 also comprises a rubber platen 21 whose ends are rotatably supported on the first mounting plate 13 and the second mounting plate 15. The rubber platen 21 is rotatably supported at the front end of the first mounting plate 13 and the second mounting plate 15 adjacent to the outside of the medical monitoring device by bearings, and a first pulley 23 is fixedly mounted at one end of the rubber platen 21. The movable frame assembly 9 also comprises an electric motor 25, which is spaced apart from the rubber platen 21 along a longitudinal direction of the first mounting plate 13 and mounted on the first mounting plate 13, and a second pulley (invisible in the drawings) is mounted on a rotating shaft of the electric motor 25. A timing belt 27 connects the first pulley 23 and the second pulley so that the electric motor drives the rubber platen 21 to rotate by means of the second pulley, the timing belt 27, and the first pulley 23 when the electric motor operates. Thus, according to the present invention, the rubber platen 21, the electric motor 25, and the timing belt 27 connecting the rubber platen 21 and the electric motor 25 are all mounted on the frame 11. In this way, even though the movable frame assembly 9 is pulled out of the housing of the medical monitoring device to pick up and add the print paper and then is inserted into the housing of the medical monitoring device 13, the rubber platen 21, the electric motor 25, and the timing belt 27 move together with the frame but their relative positions remain unchanged, thus ensuring that they are positioned accurately, which in turn allows the printing mechanism to operate smoothly without generating noise or generating significantly reduced noise.

Preferably, the electric motor 25 is disposed at a rear end of the first mounting plate 13 adjacent to the inside of the medical monitoring device, which allows the movable frame assembly 9 to be formed with a substantially uniform thickness and thus have a relatively flat and regular shape. As a result, the printing mechanism may have a relatively compact and regular layout. More preferably, the first pulley 23, the second pulley, and the electric motor 25 are all provided adjacent to the first mounting plate 13, thus making the overall structure more compact and occupying as little space as possible. The first mounting plate 13 and the second mounting plate 15 are sized such that the rubber platen 21, the first pulley on the rubber platen 21, the timing belt 27, the electric motor 25, and the second pulley on the electric motor 25 are all located inside of at least one of the first mounting plate 13 and the second mounting plate 15, ensuring that no moving component protrudes out of the frame 11 or is exposed on the outside of the frame 11. Although the first mounting plate 13 and the second mounting plate 15 may have the same length, in the preferred embodiment the length of the second mounting plate 15 may be shorter than that of the first mounting plate 13 because the second mounting plate 15 does not have to support the electric motor 25, which reduces the amount of material used and lowers the weight of the frame 11. Although in the above preferred embodiment the driving mechanism for driving the rubber platen to rotate comprises the electric motor, the first pulley, the second pulley and the timing belt, it should be understood that the driving mechanism for driving the rubber platen to rotate may be of other construction, including for example an electric motor mounted on the frame, which drives the rubber platen also mounted on the frame by gear engagement.

A first guide slot 29 and a second guide slot 31 extending along the longitudinal direction are formed in the first mounting plate 13 and the second mounting plate 15 respectively. Two ends 7a, 7b of the printing head mounting portion 7 are movably supported in the first guide slot 29 and the second guide slot 31 respectively.

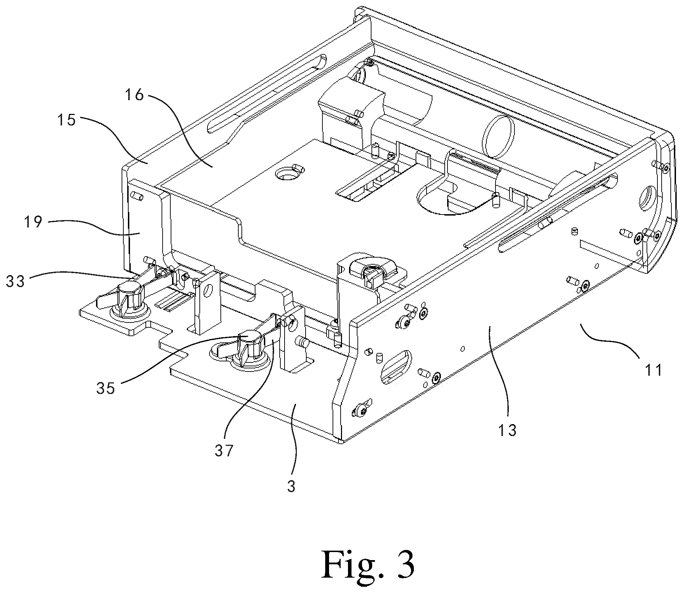

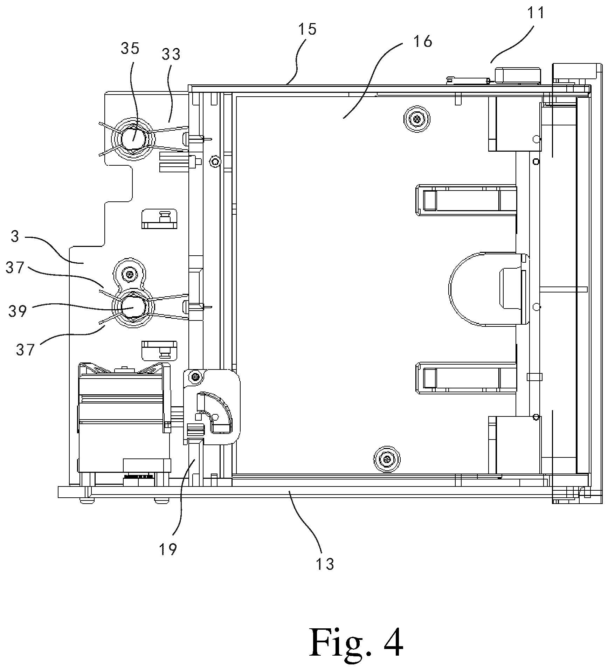

FIG. 3 shows schematically in a perspective view the printing mechanism for the medical monitoring device according to the present invention, wherein a support arm, a printing head mounting portion and a rubber platen have been removed to show clearly a print paper drawer, FIG. 4 is a top view of the structure shown in FIG. 3. A print paper drawer 16 for holding the print papers 18 is clearly shown in FIG. 3. The print paper drawer 16 may be molded from plastic, and the print paper drawer 16 is fixedly supported on the frame 11, for example, by snap, screw or other fastening means and may move along with the frame 11 or the movable frame assembly 9.

The printing mechanism 1 for the medical monitoring device according to the present invention further comprises a locking mechanism comprising a first locking member 33 provided on the outside of the second connecting plate 19 of the frame 11 (i.e., at a rear side of the frame 11 facing the inside of the housing of the medical monitoring device), and a second locking member 35 provided on the support plate 3. When the frame 11 provided with the print paper drawer 16 is pushed into the housing of the medical monitoring device, the first locking member 33 and the second locking member 35 can be engaged together under external pushing force, thereby locking the movable frame assembly 9 and the print paper drawer 16 in place in the housing of the medical monitoring device. When the frame 11 provided with the print paper drawer 16 is pulled outwardly from the housing of the medical monitoring device, the first locking member 33 and the second locking member 35 may be disengaged from each other under external pulling force, thereby pulling the movable frame assembly 9 and the print paper drawer 16 outwardly from the housing of the medical monitoring device.

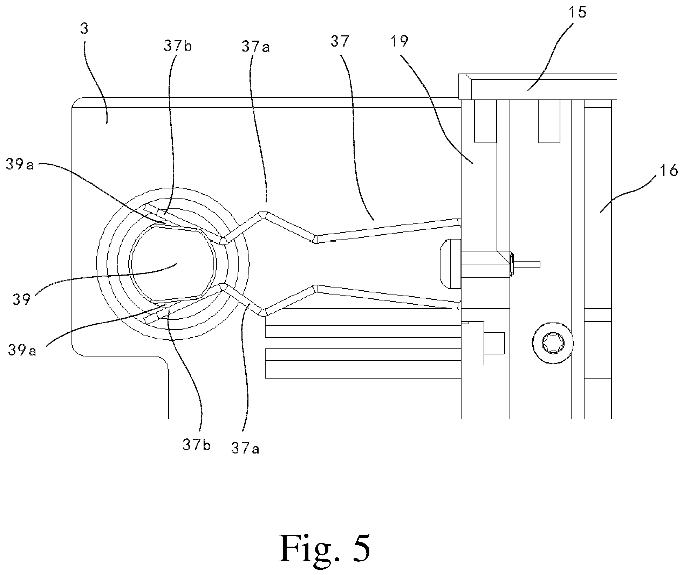

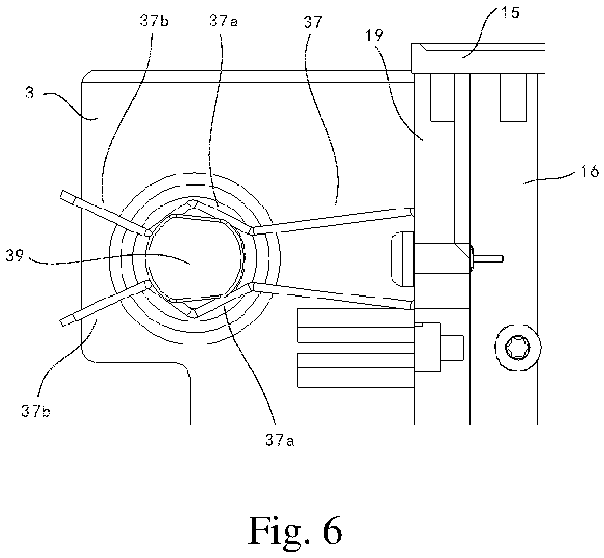

In the preferred embodiment as shown in FIGS. 3 and 4, the first locking member 33 comprises a pair of resilient clamping claws 37 disposed opposite to each other, and the resilient clamping claws 37 tend to clamp together to each other. The second locking member 35 comprises a locking post 39 disposed on the support plate 3 correspondingly to the resilient clamping claws 37. When the frame 11 provided with the print paper drawer 16 is pushed into the housing of the medical monitoring device, the resilient clamping claws 37 open under an external pushing force to receive the locking post 39 and subsequently clamp the locking post 39 tightly, thereby locking the print paper drawer 16 in place in the housing of the medical monitoring device, as shown in FIGS. 5 and 6. When the frame 11 provided with the print paper drawer 16 is pulled outwardly from the housing of the medical monitoring device, the resilient clamping claws 37 are disengaged from the locking post 39 under an external pulling force, thereby pulling the print paper drawer 16 outwardly from the housing of the medical monitoring device.

In order to reliably clamp the locking post 39 by the pair of resilient clamping claws 37, each of the resilient clamping claws 37 is formed with a wrapping portion 37a which is bent outwardly and subsequently bent inwardly. Two oppositely arranged wrapping portions 37a preferably form a () or < > shape to hold the locking post 39. Further, each of the resilient clamping claws 37 may further comprises a guiding portion 37b that is bent outwardly again from the wrapping portion 37a. Two oppositely arranged guiding portions 37b are preferably in a trumpet shape to guide the locking post 39 into a space between the two wrapping portions 37a. Although the locking post 39 may be in a cylindrical shape, two sides of the locking post 39 are preferably beveled and two planes 39a formed by beveled sides converge toward the print paper drawer 16, which further helps to guide the locking post 39 into engagement with the pair of resilient clamping claws 37.

According to the locking mechanism of the preferred embodiment of the present invention, when the print paper drawer 16 is about to be fully closed, the inner walls of the wrapping portions 37a of the resilient clamping claws 37 exert a pushing force on the locking post 39, which helps to push the print paper drawer 16 into the housing. After the print paper drawer 16 is fully closed, the resilient clamping claws 37 reset and generate a "click" sound to provide a feedback to the user that the print paper drawer 16 is fully closed.

In the preferred embodiment shown in Figures 3 and 4, there are two pairs of resilient clamping claws 37 and two corresponding locking posts 39. It is understood that it is possible to have only one or more pairs of resilient clamping claws 37 and corresponding locking post 39. It is also feasible that the pairs of resilient clamping claws are disposed on the support plate 3 while the locking posts 39 are disposed on the frame 11.

In a normal state, the movable frame assembly 9 is inserted into the space between the support plate 3 and the support arm 5 in the housing of the medical monitoring device such that the printing head on the printing head mounting portion 7 is in contact with the rubber platen 21 under pressure. The print paper 16 may pass between the rubber platen 21 and the printing head. When the printing mechanism operates, the electric motor 25 drives the rubber platen 21 to rotate by means of the second pulley, the electric motor 25 and the first pulley 23, thereby dragging thermal print paper past the printing head to record a curve characterizing fetal vital characteristics on the print paper.

When the movable frame assembly 9 is pulled outwardly to pick up and add the print papers, the resilient clamping claws 37 disengages from the locking posts 39 under external pulling force, thereby moving the print paper drawer to the outside of the housing of the medical monitoring device to pick up and add the print paper. After picking up and adding the print paper, the resilient clamping claws 37 open under external pushing force to receive the locking post 39 and subsequently clamp the locking post 39 tightly, thereby locking the print paper drawer 16 in place in the housing of the medical monitoring device.

The printing mechanism according to the present invention has fewer parts and is simpler in design and assembly. And, in use, the user may perform the opening and closing operation of the print paper drawer with only one hand without any additional pressing operation or great effort. Further, the print paper drawer may be pushed or pulled steadily without any wobbling, and achieve a stable and reliable locking.

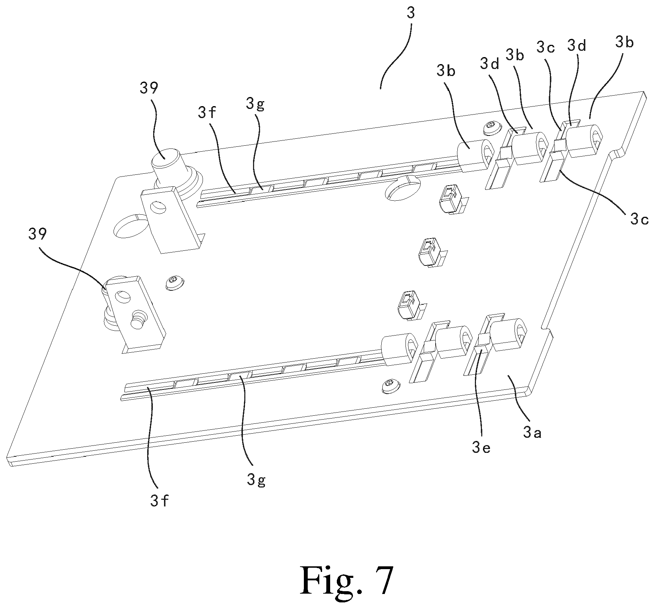

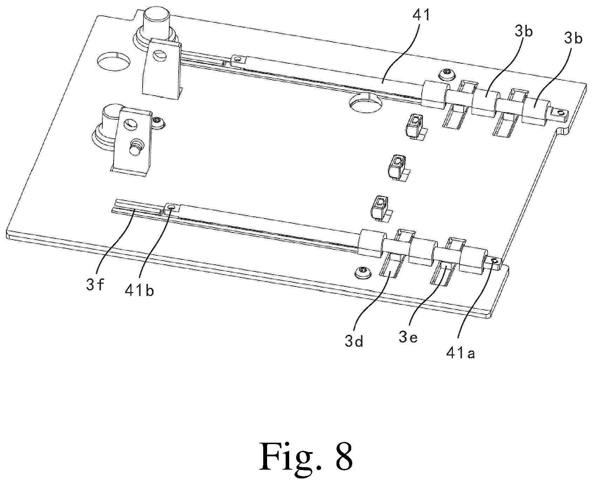

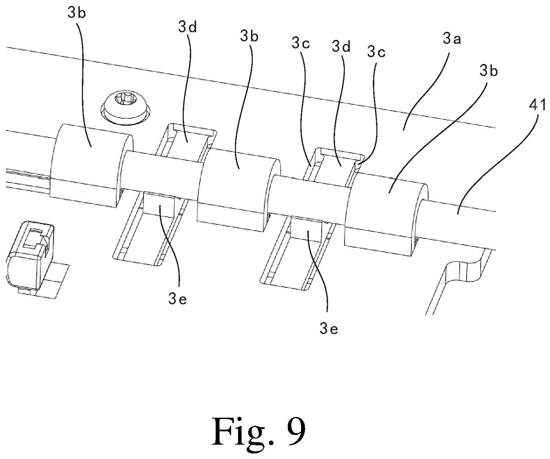

FIG. 7 schematically shows in a perspective view a support plate of a printing mechanism for a medical monitoring device according to the present invention. As shown in FIG. 7, the support plate 3 comprises a substantially plate-like body 3a made of metal or plastic, and at least one set of spaced sleeves 3b are provided on the top surface of the body 3a (i.e., the surface for supporting the movable frame assembly 9) in a movement direction of the movable frame assembly 9 so that the holes defined by each set of sleeves 3b substantially align with each other so as to receive a guide rod 41 (as shown in FIGS. 8 and 9). A bottom surface of the holes defined by the sleeves 3b facing the body 3a may be open. In the preferred embodiment, two sets of spaced sleeves are shown but it should be understood that only one set of spaced sleeves or more sets of spaced sleeves may be provided. Two elongated openings 3c are formed at positions between adjacent sleeves 3b of each set of sleeves so as to define an elastic beam 3d between the two elongated openings 3c. That is to say, by forming two elongated openings 3c, a portion of the body located between the two elongated openings 3c has a certain elasticity so that it may deform elastically in a direction perpendicular to the body 3a as desired. A V-shaped, U-shaped or semi-circular damping bracket 3e may be provided on each of the elastic beams 3d. The damping bracket is usually made of glass fiber reinforced nylon or other plastic material. Although in the preferred embodiment the elastic beam 3d is formed between adjacent sleeves 3b of each set of sleeves and the damping bracket 3e is provided on the elastic beam 3d, it should be understood that it is also feasible not to form the elastic beam 3d.

As shown in FIGS. 8 and 9, the guide rod 41 passes through the holes of each set of spaced sleeves 3b and the guide rod 41 is supported on the damping bracket 3e on each elastic beam 3d, and the guide rod 41 is subsequently secured to the bottom of the frame 11 of the movable frame assembly 9 by screws passing through the holes 41a, 41b at each end of the guide rod 41 so that the guide rod 41 may guide the movable frame assembly 9 and move along with the movable frame assembly 9. As shown in FIG. 2, the guide rod 41 is preferably secured to the first connecting plate 17 and the second connecting plate 19. In this way, by the sleeves 3b on the support plate 3, the guide rod 41 can guide the frame 11 or the movable frame assembly 9 to move. The guide rod 41 squeezes the damping brackets 3e when the guide rod 41 comes into contact with the damping bracket 3e on each elastic beam 3d, which in turn causes the elastic beam 3d to elastically deform downward perpendicularly to the movement direction of the guide rod 41. The damping brackets 3e thus exert an upward reaction force onto the guide rod 41 perpendicularly to the movement direction of the guide rod 41. The reaction force will not only generate a beneficial friction force between the damping brackets 3e and the guide rod 41 but also cause the guide rod 41 to abut closely against the sleeves 3b to generate another beneficial frictional force. These beneficial frictions provide a beneficial damping force for the movement of the movable frame assembly 9 (i.e., the print paper drawer), which not only provides a good controllability when the user pushes and pulls the print paper drawer, but also prevents the print paper drawer from wobbling, thereby ensuring that the print paper drawer is positioned with high accuracy and is opened and closed smoothly when the printing mechanism operates in the horizontally arranged state or the vertically hung state.

In order to easily pull the print paper drawer out, the number of sleeves in each set of sleeves is not too much. In order to further guide and limit the guide rod 41, a guide groove 3f is provided on the body 3a of the support plate 3 at a portion where the guide rod 41 passes but no sleeve is provided. As a result, the guide rod 41 may be partially accommodated in the guide groove 3f when it passes the guide groove 3f.

Spaced through-holes 3g may be formed in the support plate 3 at a position where the guide groove 3f is provided. In the preferred embodiment, four through-holes 3g are shown at the position where the guide groove 3f is provided. It should be understood that the number of through-holes may be more or less than four. As mentioned above, the bottom surface of the holes defined by the sleeves 3b facing the body 3a may be open. In this case, it means that in the preferred embodiment seven through-holes are respectively formed in the body 3a at the position where each guide rod 41 passes.

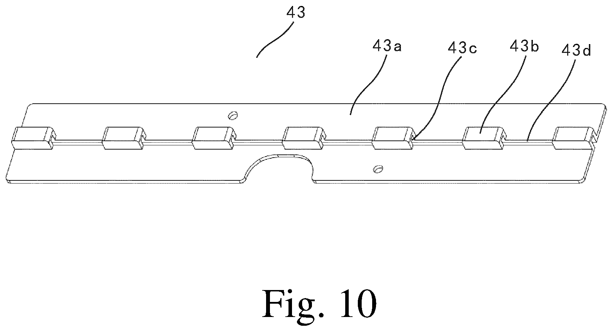

FIG. 10 schematically shows in a perspective view a friction strip of a printing mechanism for a medical monitoring device according to the present invention. As shown in FIG. 10, each friction strip 43 comprises a planar-shaped carrier 43a and a projecting pad 43b formed at the center of the carrier 43a. The number of the pads 43b corresponds to the number of the through-holes on the portion of the body 3a through which the guide rod 41 passes. A cross-sectional shape and size of the pad 43b are substantially the same as the shape and size of the through-hole 3g so that the pad 43b passes through the through-hole 3g from the bottom of the body 3a and protrudes slightly from the through-hole 3g toward the top of the body 3a. The friction strip 43 may be made of a resilient material such as rubber. The guide rod 41 squeezes the pads 43b of the friction strip 43 when it comes into contact with the pad 43b, thereby generating another beneficial frictional force. This beneficial frictional force further ensures that the print paper drawer is positioned with high accuracy when the printing mechanism operates in the horizontally arranged state or the vertically hung state. In order to facilitate the elastic deformation of the pad 43b and to ensure the proper friction, a hollow section 43c may be formed below the top surface of the pads 43b. In the preferred embodiment, a trench 43d is formed in the carrier 43a of the friction strip 43 to connect the various hollow sections 43c.

According to the present invention, by providing the guide groove and/or the friction strip with the pad, the positioning accuracy and the smoothness of the opening and closing of the print paper drawer when the printing mechanism operates in the horizontally arranged state or the vertically hung state can be further improved.

Although the present invention has been described in detail in connection with preferred embodiments, it should be understood that such detailed description is intended only to illustrate the present invention and does not constitute any limit to the present invention. For example, the first locking member may be provided as a shaft, the second locking member may be provided as a sleeve for receiving the shaft, and the shaft and the sleeve may engage releasably with each other by friction force. Thus, the present invention is defined in the appended claims.

## Claims

1. A printing mechanism for a medical monitoring device comprising:
a support plate (3);
a movable frame assembly (9) disposed movably on the support plate (3), the movable frame assembly (9) comprising a frame (11); and
a print paper drawer (16) for holding the print papers, the print paper drawer (16) being mounted on the frame (11) to move along with the movable frame assembly;
**characterized in that** the printing mechanism further comprises:
at least one set of spaced sleeves (3b) provided on a top surface of a body (3a) of the support plate (3) in a movement direction of the movable frame assembly (9); and
a guide rod (41) passing through holes defined by each set of the spaced sleeves (3b) and secured to a bottom of the frame (11) at two ends, the guide rod (41) guiding the movable frame assembly (9) and moving along with the movable frame assembly (9);
wherein a damping bracket (3e) is provided between adjacent sleeves (3b) of each set of the spaced sleeves, the damping bracket (3e) contacts the guide rod (41) and exerts an upward force onto the guide rod (41) perpendicularly to a movement direction of the guide rod.

2. A printing mechanism for a medical monitoring device as claimed in claim 1, wherein two elongated openings (3c) are formed between adjacent sleeves (3b) of each set of the spaced sleeves so as to define an elastic beam (3d) between the two elongated openings (3c), and the damping bracket (3e) is provided on the elastic beam (3d).

3. A printing mechanism for a medical monitoring device as claimed in claim 1, wherein the damping bracket (3e) is V-shaped, U-shaped or semi-circular.

4. A printing mechanism for a medical monitoring device as claimed in claim 1, wherein a guide groove (3f) is provided on the body (3a) at a portion where the guide rod 41 passes but no sleeve is provided, and the guide rod (41) is partially accommodated in the guide groove (3f) when it passes the guide groove (3f).

5. A printing mechanism for a medical monitoring device as claimed in claim 4, wherein spaced through-holes (3g) are formed in the body (3a) at a position where the guide groove (3f) is provided, the printing mechanism further comprises a friction strip (43) comprising a planar-shaped carrier (43a) and a projecting pad (43b) formed at the center of the carrier (43a), the pad (43b) passes through the through-hole (3g) from the bottom of the body (3a) and protrudes toward a top of the body (3a) so that the guide rod (41) squeezes the pad (43b) when it comes into contact with the pad (43b).

6. A printing mechanism for a medical monitoring device as claimed in claim 5, wherein a bottom surface of the holes defined by the sleeves (3b) facing the body (3a) is open, and the pad (43b) passes through a through-hole below the sleeve (3b).

7. A printing mechanism for a medical monitoring device as claimed in claim 4, wherein a hollow section (43c) is formed below a top surface of the pad (43b).

8. A printing mechanism for a medical monitoring device as claimed in claim 1, wherein the printing mechanism further comprises:
a support arm (5) disposed on the support plate (3), the support arm (5) provided with a printing head thereon, the movable frame assembly (9) further comprising a rubber platen (21) mounted at a front end of the frame (11) adjacent to the outside of the medical monitoring device to be in contact with the printing head under pressure; and
a driving mechanism for driving the rubber platen (21) to rotate;
wherein the movable frame assembly (9) is disposed between the support plate (3) and the support arm (5).

9. A printing mechanism for a medical monitoring device as claimed in claim 8, wherein the printing mechanism further comprises a locking mechanism comprising a pair of resilient clamping claws (37) disposed opposite to each other at a rear end of the frame (11) and a locking post (39) disposed on the support plate (3) correspondingly to the resilient clamping claws (37), the resilient clamping claws (37) tend to clamp together, the resilient clamping claws (37) open under an external pushing force to receive the locking post (39) and clamp the locking post (39) tightly, and the resilient clamping claws (37) are disengaged from the locking post (39) under an external pulling force.

10. A printing mechanism for a medical monitoring device as claimed in claim 8, wherein the driving mechanism comprises a first pulley (23) fixedly mounted at one end of the rubber platen (21), an electric motor (25) mounted on the frame (11), a second pulley mounted on a rotating shaft of the electric motor (25), and a timing belt (27) for connecting the first pulley (29) and the second pulley so as to drive the rubber platen (21) to rotate when the electric motor (25) operates, and the electric motor (25) is disposed at a rear end of the frame (11) adjacent to the inside of the medical monitoring device.

11. A printing mechanism for a medical monitoring device as claimed in claim 8, wherein the support arm (5) is L-shaped, the support arm (5) is rotatably mounted on the support plate (3) at one end (5a) and provided with a printing head mounting portion (7) at the other end (5b), and the printing head is mounted on the printing head mounting portion (7).

12. A printing mechanism for a medical monitoring device as claimed in claim 11, wherein the frame (11) comprises a first mounting plate (13) and a second mounting plate (15) disposed opposite to each other, and a first connecting plate (17) and a second connecting plate (19) connecting fixedly the first mounting plate (13) and the second mounting plate (15) together, a first guide slot (29) extending along a longitudinal direction is formed in the first mounting plate (13) and a second guide slot (31) extending along the longitudinal direction is formed in the second mounting plate (15), and two ends (7a, 7b) of the printing head mounting portion (7) are movably supported in the first guide slot (29) and the second guide slot (31).

13. A printing mechanism for a medical monitoring device as claimed in claim 1, wherein the medical monitoring device is a fetal monitor.

14. A medical monitoring device, wherein the medical monitoring device comprises a printing mechanism for a medical monitoring device as claimed in any one of claims 1-13.

## Patentansprüche

1. Druckmechanismus für eine medizinische Überwachungsvorrichtung, umfassend:
eine Trägerplatte (3);
eine bewegliche Rahmenanordnung (9), die beweglich auf der Trägerplatte (3) angeordnet ist, wobei die bewegliche Rahmenanordnung (9) einen Rahmen (11) umfasst; und
eine Druckpapierschublade (16) zum Halten der Druckpapiere, wobei die Druckpapierschublade (16) auf dem Rahmen (11) montiert ist, um sich zusammen mit der beweglichen Rahmenanordnung zu bewegen;
**dadurch gekennzeichnet, dass** der Druckmechanismus weiter umfasst:
mindestens einen Satz beabstandeter Hülsen (3b), die auf einer oberen Oberfläche eines Körpers (3a) der Trägerplatte (3) in einer Bewegungsrichtung der beweglichen Rahmenanordnung (9) bereitgestellt sind; und
eine Führungsstange (41), die durch Löcher verläuft, die durch jeden Satz der beabstandeten Hülsen (3b) definiert sind, und an zwei Enden an einer Unterseite des Rahmens (11) gesichert ist, wobei die Führungsstange (41) die bewegliche Rahmenanordnung (9) führt und sich zusammen mit der beweglichen Rahmenanordnung (9) bewegt;
wobei zwischen benachbarten Hülsen (3b) jedes Satzes beabstandeter Hülsen eine Dämpfungshalterung (3e) bereitgestellt ist, die Dämpfungshalterung (3e) die Führungsstange (41) berührt und senkrecht zu einer Bewegungsrichtung der Führungsstange eine nach oben gerichtete Kraft auf die Führungsstange (41) ausübt.

2. Druckmechanismus für eine medizinische Überwachungsvorrichtung nach Anspruch 1, wobei zwischen benachbarten Hülsen (3b) jedes Satzes der beabstandeten Hülsen zwei längliche Öffnungen (3c) gebildet sind, um zwischen den beiden länglichen Öffnungen (3c) einen elastischen Balken (3d) zu definieren, und die Dämpfungshalterung (3e) auf dem elastischen Balken (3d) bereitgestellt ist.

3. Druckmechanismus für eine medizinische Überwachungsvorrichtung nach Anspruch 1, wobei die Dämpfungshalterung (3e) V-förmig, U-förmig oder halbkreisförmig ist.

4. Druckmechanismus für eine medizinische Überwachungsvorrichtung nach Anspruch 1, wobei an dem Körper (3a) an einem Abschnitt, wo die Führungsstange (41) verläuft, jedoch keine Hülse bereitgestellt ist, eine Führungsnut (3f) bereitgestellt ist, und die Führungsstange (41) teilweise in der Führungsnut (3f) untergebracht ist, wenn sie in der Führungsnut (3f) verläuft.

5. Druckmechanismus für eine medizinische Überwachungsvorrichtung nach Anspruch 4, wobei im Körper (3a) an einer Position, an der die Führungsnut (3f) bereitgestellt ist, beabstandete Durchgangslöcher (3g) gebildet sind, der Druckmechanismus weiter einen Reibungsstreifen (43) umfasst, der einen eben geformten Träger (43a) und ein hervorstehendes Kissen (43b) umfasst, das in der Mitte des Trägers (43a) gebildet ist, das Kissen (43b) von der Unterseite des Körpers (3a) durch das Durchgangsloch (3g) verläuft und in Richtung einer Oberseite des Körpers (3a) hervorsteht, sodass die Führungsstange (41) das Kissen (43b) zusammendrückt, wenn sie mit dem Kissen (43b) in Kontakt kommt.

6. Druckmechanismus für eine medizinische Überwachungsvorrichtung nach Anspruch 5, wobei eine dem Körper (3a) zugewandte untere Oberfläche der durch die Hülsen (3b) definierten Löcher offen ist und das Kissen (43b) durch ein Durchgangsloch unter der Hülse (3b) verläuft.

7. Druckmechanismus für eine medizinische Überwachungsvorrichtung nach Anspruch 4, wobei unter einer oberen Oberfläche des Kissens (43b) ein hohler Abschnitt (43c) gebildet ist.

8. Druckmechanismus für eine medizinische Überwachungsvorrichtung nach Anspruch 1, wobei der Druckmechanismus weiter umfasst:
einen auf der Trägerplatte (3) angeordneten Trägerarm (5), wobei der Trägerarm (5) mit einem Druckkopf bereitgestellt ist, wobei die bewegliche Rahmenanordnung (9) weiter eine Gummiplatte (21) umfasst, die an einem vorderen Ende des Rahmens (11) neben der Außenseite der medizinischen Überwachungsvorrichtung montiert ist, um unter Druck mit dem Druckkopf in Kontakt zu sein; und
einen Antriebsmechanismus zum Antreiben der Gummiplatte (21), um sich zu drehen;
wobei die bewegliche Rahmenanordnung (9) zwischen der Trägerplatte (3) und dem Trägerarm (5) angeordnet ist.

9. Druckmechanismus für eine medizinische Überwachungsvorrichtung nach Anspruch 8, wobei der Druckmechanismus weiter einen Verriegelungsmechanismus umfasst, der ein Paar elastischer Klemmklauen (37), die einander gegenüberliegend an einem hinteren Ende des Rahmens (11) angeordnet sind, und einen Verriegelungspfosten (39) umfasst, der entsprechend den elastischen Klemmklauen (37) auf der Trägerplatte (3) angeordnet ist, wobei die elastischen Klemmklauen (37) dazu neigen, sich zusammenzuklemmen, wobei sich die elastischen Klemmklauen (37) unter einer externen Schubkraft öffnen, um den Verriegelungspfosten (39) aufzunehmen und den Verriegelungspfosten (39) fest zu klemmen, und die elastische Klemmklauen (37) sich unter einer externen Zugkraft von dem Verriegelungspfosten (39) lösen.

10. Druckmechanismus für eine medizinische Überwachungsvorrichtung nach Anspruch 8, wobei der Antriebsmechanismus eine erste Riemenscheibe (23), die fest an einem Ende der Gummiplatte (21) montiert ist, einen am Rahmen (11) montierten Elektromotor (25), eine zweite Riemenscheibe, die auf einer Drehwelle des Elektromotors (25) montiert ist, und einen Zahnriemen (27) zum Verbinden der ersten Riemenscheibe (29) und der zweiten Riemenscheibe umfasst, um die Gummiplatte (21) anzutreiben, um sich zu drehen, wenn der Elektromotor (25) in Betrieb ist, und der Elektromotor (25) an einem hinteren Ende des Rahmens (11) neben der Innenseite der medizinischen Überwachungsvorrichtung angeordnet ist.

11. Druckmechanismus für eine medizinische Überwachungsvorrichtung nach Anspruch 8, wobei der Trägerarm (5) L-förmig ist, der Trägerarm (5) an einem Ende (5a) drehbar auf der Trägerplatte (3) montiert ist und am anderen Ende (5b) mit einem Druckkopfmontageabschnitt (7) bereitgestellt ist und der Druckkopf auf dem Druckkopfmontageabschnitt (7) montiert ist.

12. Druckmechanismus für eine medizinische Überwachungsvorrichtung nach Anspruch 11, wobei der Rahmen (11) eine erste Montageplatte (13) und eine zweite Montageplatte (15) umfasst, die einander gegenüberliegend angeordnet sind, und eine erste Verbindungsplatte (17) und eine zweite Verbindungsplatte (19), welche die erste Montageplatte (13) und die zweite Montageplatte (15) fest miteinander verbinden, wobei in der ersten Montageplatte (13) ein erster Führungsschlitz (29) gebildet ist, der sich entlang einer Längsrichtung erstreckt, und in der zweiten Montageplatte (15) ein zweiter Führungsschlitz (31) gebildet ist, der sich entlang der Längsrichtung erstreckt, und zwei Enden (7a, 7b) des Druckkopfmontageabschnitts (7) beweglich in dem ersten Führungsschlitz (29) und dem zweiten Führungsschlitz (31) getragen werden.

13. Druckmechanismus für eine medizinische Überwachungsvorrichtung nach Anspruch 1, wobei die medizinische Überwachungsvorrichtung ein Fetal-Monitor ist.

14. Medizinische Überwachungsvorrichtung, wobei die medizinische Überwachungsvorrichtung einen Druckmechanismus für eine medizinische Überwachungsvorrichtung nach einem der Ansprüche 1-13 umfasst.

## Revendications

1. Mécanisme d'impression destiné à un dispositif de surveillance médicale comprenant :
une plaque de support (3) ;
un ensemble cadre mobile (9) disposé de manière mobile sur la plaque de support (3), l'ensemble cadre mobile (9) comprenant un cadre (11) ; et
un tiroir à papier d'impression (16) pour contenir les papiers d'impression, le tiroir à papier d'impression (16) étant monté sur le cadre (11) pour se déplacer avec l'ensemble cadre mobile ;
**caractérisé en ce que** le mécanisme d'impression comprend en outre :
au moins un ensemble de manchons (3b) espacés prévus sur une surface supérieure d'un corps (3a) de la plaque de support (3) dans une direction de déplacement de l'ensemble cadre mobile (9) ; et
une tige de guidage (41) traversant des trous définis par chaque ensemble de manchons (3b) espacés et fixée à un fond du cadre (11) à deux extrémités, la tige de guidage (41) guidant l'ensemble cadre mobile (9) et se déplaçant avec l'ensemble cadre mobile (9) ;
dans lequel un support d'amortissement (3e) est prévu entre des manchons (3b) adjacents de chaque ensemble de manchons espacés, le support d'amortissement (3e) entre en contact avec la tige de guidage (41) et exerce une force vers le haut sur la tige de guidage (41) perpendiculairement à une direction de déplacement de la tige de guidage.

2. Mécanisme d'impression destiné à un dispositif de surveillance médicale selon la revendication 1, dans lequel deux ouvertures allongées (3c) sont formées entre des manchons (3b) adjacents de chaque ensemble de manchons espacés de manière à définir une barre élastique (3d) entre les deux ouvertures allongées (3c), et le support d'amortissement (3e) est prévu sur la barre élastique (3d).

3. Mécanisme d'impression destiné à un dispositif de surveillance médicale selon la revendication 1, dans lequel le support d'amortissement (3e) est en forme de V, en forme de U ou semi-circulaire.

4. Mécanisme d'impression destiné à un dispositif de surveillance médicale selon la revendication 1, dans lequel une rainure de guidage (3f) est prévue sur le corps (3a) au niveau d'une partie où passe la tige de guidage (41), mais aucun manchon n'est prévu, et la tige de guidage (41) est partiellement logée dans la rainure de guidage (3f) lorsqu'elle passe la rainure de guidage (3f).

5. Mécanisme d'impression destiné à un dispositif de surveillance médicale selon la revendication 4, dans lequel des trous traversants (3g) espacés sont formés dans le corps (3a) à une position où la rainure de guidage (3f) est prévue, le mécanisme d'impression comprend en outre une bande de frottement (43) comprenant un support de forme plane (43a) et un tampon en saillie (43b) formé au centre du support (43a), le tampon (43b) passe à travers le trou traversant (3g) depuis le bas du corps (3a) et fait saillie vers le haut du corps (3a) de sorte que la tige de guidage (41) comprime le tampon (43b) lorsqu'elle entre en contact avec le tampon (43b).

6. Mécanisme d'impression destiné à un dispositif de surveillance médicale selon la revendication 5, dans lequel une surface inférieure des trous définis par les manchons (3b) faisant face au corps (3a) est ouverte, et le tampon (43b) passe à travers un trou traversant sous le manchon (3b).

7. Mécanisme d'impression destiné à un dispositif de surveillance médicale selon la revendication 4, dans lequel une section creuse (43c) est formée sous une surface supérieure du tampon (43b).

8. Mécanisme d'impression destiné à un dispositif de surveillance médicale selon la revendication 1, dans lequel le mécanisme d'impression comprend en outre :
un bras de support (5) disposé sur la plaque de support (3), le bras de support (5) étant pourvu d'une tête d'impression, l'ensemble cadre mobile (9) comprenant en outre un plateau en caoutchouc (21) monté au niveau d'une extrémité avant du cadre (11) de manière adjacente à l'extérieur du dispositif de surveillance médicale pour entrer en contact avec la tête d'impression sous pression ; et
un mécanisme d'entraînement pour entraîner en rotation le plateau en caoutchouc (21) ;
dans lequel l'ensemble cadre mobile (9) est disposé entre la plaque de support (3) et le bras de support (5).

9. Mécanisme d'impression destiné à un dispositif de surveillance médicale selon la revendication 8, dans lequel le mécanisme d'impression comprend en outre un mécanisme de verrouillage comprenant une paire de griffes de serrage élastiques (37) disposées l'une en face de l'autre à une extrémité arrière du cadre (11) et un montant de verrouillage (39) disposé sur la plaque de support (3) en correspondance avec les griffes de serrage élastiques (37), les griffes de serrage élastiques (37) ont tendance à se serrer ensemble, les griffes de serrage élastiques (37) s'ouvrent sous une force de poussée externe pour recevoir le montant de verrouillage (39) et serrent le montant de verrouillage (39) fermement, et les griffes de serrage élastiques (37) sont désengagées du montant de verrouillage (39) sous une force de traction externe.

10. Mécanisme d'impression destiné à un dispositif de surveillance médicale selon la revendication 8, dans lequel le mécanisme d'entraînement comprend une première poulie (23) montée de manière fixe à une extrémité du plateau en caoutchouc (21), un moteur électrique (25) monté sur le cadre (11), une seconde poulie montée sur un arbre rotatif du moteur électrique (25), et une courroie de distribution (27) pour relier la première poulie (29) et la seconde poulie de manière à entraîner le plateau en caoutchouc (21) en rotation lorsque le moteur électrique (25) fonctionne, et le moteur électrique (25) est disposé à une extrémité arrière du cadre (11) de manière adjacente à l'intérieur du dispositif de surveillance médicale.

11. Mécanisme d'impression destiné à un dispositif de surveillance médicale selon la revendication 8, dans lequel le bras de support (5) est en forme de L, le bras de support (5) est monté de manière rotative sur la plaque de support (3) à une extrémité (5a) et pourvu d'une partie de montage de tête d'impression (7) à l'autre extrémité (5b), et la tête d'impression est montée sur la partie de montage de tête d'impression (7).

12. Mécanisme d'impression destiné à un dispositif de surveillance médicale selon la revendication 11, dans lequel le cadre (11) comprend une première plaque de montage (13) et une seconde plaque de montage (15) disposées en face l'une de l'autre, et une première plaque de liaison (17) et une seconde plaque de liaison (19) reliant de manière fixe la première plaque de montage (13) et la seconde plaque de montage (15) ensemble, une première fente de guidage (29) s'étendant suivant une direction longitudinale est formée dans la première plaque de montage (13) et une seconde fente de guidage (31) s'étendant suivant la direction longitudinale est formée dans la seconde plaque de montage (15), et deux extrémités (7a, 7b) de la partie de montage de la tête d'impression (7) sont soutenues de manière mobile dans la première fente de guidage (29) et la seconde fente de guidage (31).

13. Mécanisme d'impression destiné à un dispositif de surveillance médicale selon la revendication 1, dans lequel le dispositif de surveillance médicale est un moniteur fœtal.

14. Dispositif de surveillance médicale, dans lequel le dispositif de surveillance médicale comprend un mécanisme d'impression destiné à un dispositif de surveillance médicale selon l'une quelconque des revendications 1-13.
